(19) Europäisches Patentamt / European Patent Office / Office européen des brevets

(11) **EP 3 671 179 B1**

(12) **FASCICULE DE BREVET EUROPEEN**

(45) Date de publication et mention
de la délivrance du brevet:
**30.10.2024 Bulletin 2024/44**

(21) Numéro de dépôt: **19218622.9**

(22) Date de dépôt: **20.12.2019**

(51) Classification Internationale des Brevets (IPC):
**G01N 15/02** *(2024.01)* **G01N 15/14** *(2024.01)*
**G01N 21/47** *(2006.01)* **G01N 21/53** *(2006.01)*

(52) Classification Coopérative des Brevets (CPC):
**G01N 15/0211; G01N 15/1436; G01N 15/1459;**
G01N 2021/4716

(54) **DÉTECTEUR OPTIQUE DE PARTICULES**

OPTISCHER TEILCHENDETEKTOR

OPTICAL PARTICLE SENSOR

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.12.2018 FR 1874073**

(43) Date de publication de la demande:
**24.06.2020 Bulletin 2020/26**

(73) Titulaire: **Commissariat à l'Energie Atomique et
aux Energies
Alternatives
75015 Paris (FR)**

(72) Inventeur: **JOBERT, Gabriel
38054 Cedex 9 GRENOBLE (FR)**

(74) Mandataire: **Hautier IP
20, rue de la Liberté
06000 Nice (FR)**

(56) Documents cités:
WO-A1-2004/019031    WO-A1-2016/001324
US-A1- 2005 195 605    US-A1- 2010 288 921
US-A1- 2011 223 587    US-B1- 6 519 033

## Description

### DOMAINE TECHNIQUE

La présente invention concerne le domaine de la détection optique de particules en général et plus particulièrement de particules de taille micrométrique, voire nanométrique. Elle concerne également l'analyse angulaire de la diffusion d'une ou plusieurs particules.

Elle trouvera pour application particulièrement avantageuse, mais non limitative, le contrôle de la qualité de l'air, la détection d'espèces microbiologiques, la détection d'incendies, la détection de poudre d'explosifs.

### ETAT DE LA TECHNIQUE

Les particules sont des objets microscopiques solides, liquides ou solides mouillés en suspension dans l'air. Leurs tailles varient de quelques dizaines de nanomètres à quelques dizaines de micromètres. Ces particules proviennent de sources variées telles que des incendies de forêts, des sites de construction, des sites industriels, des véhicules motorisés etc.

Lorsque la concentration de ces particules dépasse un certain seuil, elles ont un impact néfaste sur l'environnement et/ou la santé. Ainsi, des états ont fixé des seuils de concentration maximale. Par exemple, l'Union Européenne permet des concentrations maximales de 50 $\mu$g/m3 pour les particules de taille comprise entre 10 $\mu$m et 2.5 $\mu$m et de 25 $\mu$g/m3 pour les particules de taille inférieure à 2.5 $\mu$m.

Il est donc nécessaire de détecter avec précision la présence et la concentration de ces particules par gamme de taille.

Différentes méthodes de détection de particules existent, telles que les méthodes de détection par gravimétrie, par ionisation, par atténuation bêta, les méthodes de détection par mesure de mobilité aérodynamique ou de mobilité électrique, les méthodes de détection optiques.

Ces dernières sont avantageusement plus simples à mettre en oeuvre, et plus répandues. La détection optique de particules se fait généralement par l'intermédiaire d'un dispositif comprenant une source de lumière, qui éclaire un canal à travers lequel passent les particules à détecter, et au moins un photodétecteur.

Si des particules sont présentes dans la zone éclairée, celles-ci vont absorber une partie de la lumière qui provient de la source et vont dévier une autre partie de cette lumière hors de la direction principale de propagation, selon le phénomène de diffusion.

Une première méthode de détection optique, dite mesure d'obscuration, consiste donc à mesurer l'absorption de la lumière à travers un nuage de particules ou une accumulation de particules. Cette mesure permet de déterminer la concentration des particules à l'aide de la loi de Beer-Lambert si on connait a priori la composition du nuage de particules.

Une deuxième méthode consiste à analyser la lumière diffusée hors de l'axe optique. L'analyse angulaire de la lumière diffusée, à partir de l'enregistrement d'un diagramme de rayonnement angulaire, permet de déterminer la forme, la taille, l'indice optique et la concentration des particules. Par exemple, la taille, l'indice de réfraction et la concentration de particules peuvent être déterminés à partir des théories de diffusion de la lumière, par exemple la théorie de Mie (Réf : Bohren et Huffmann, Absorption and scattering of light by small particles, Ed. Wiley and Sons, 1983). Pour effectuer cette analyse angulaire, on peut utiliser par exemple un dispositif comprenant un goniomètre constitué d'un photodétecteur monté sur un bras rotatif, ou un dispositif comprenant un assemblage discret de photodétecteurs distribués en dehors de l'axe optique.

Une analyse angulaire par un dispositif comprenant un goniomètre présente pour inconvénients d'être très complexe, très onéreux et peu robuste. Ainsi, il ne peut pas être transporté aisément. Par ailleurs, il ne peut pas être envisagé pour équiper des systèmes d'alarme ou de mesure à coût faible. Or, par exemple pour le domaine de la détection des incendies dans les habitations ou pour le domaine du contrôle de la qualité de l'air, il est indispensable de proposer des solutions dont les coûts sont faibles et dont la robustesse est élevée.

Le document FR3062209 A1 décrit une autre solution pour réaliser une analyse angulaire. Cette solution est basée sur un dispositif comprenant un assemblage discret de photodétecteurs formant une matrice ainsi que des surfaces réfléchissantes pour projeter sur la matrice l'image du rayonnement diffusé par les particules. En pratique, il s'avère que cette solution génère un flou angulaire sur le diagramme de rayonnement angulaire.

Cette solution génère également un bruit de mesure sur le diagramme angulaire.

L'analyse angulaire réalisée par un tel détecteur est donc bruitée et peu résolue.

Il est donc difficile avec ce type de solution d'obtenir des informations suffisamment précises et complètes sur les particules.

Le document US 2005/0195605 A1 décrit encore une autre solution permettant de détecter des particules conforme à l'état de l'art antérieur.

La présente invention a pour objectif de pallier au moins en partie les inconvénients cités ci-dessus.

Un objet de la présente invention est un détecteur optique de particules permettant d'obtenir un diagramme de rayonnement angulaire en limitant le flou angulaire.

Un autre objet de la présente invention est un détecteur optique de particules permettant d'obtenir un diagramme de rayonnement angulaire en limitant le bruit de mesure.

**RESUME**

La présente invention concerne un détecteur optique de particules tel que défini dans la revendication 1 et comprenant au moins:

- un canal destiné à recevoir un fluide comprenant au moins une particule et à recevoir au moins un rayon lumineux incident,
- un système de détection comprenant une pluralité de photodétecteurs, chaque photodétecteur étant apte à recevoir des rayons lumineux provenant du canal et diffusés par l'au moins une particule.

Conformément à l'invention, le détecteur comprend en outre un système de filtrage angulaire comprenant une pluralité de dispositifs de filtrage angulaire associés chacun à un photodétecteur, chaque dispositif de filtrage angulaire étant configuré pour filtrer angulairement les rayons lumineux provenant du canal avant leur réception par le photodétecteur auquel il est associé.

Chaque photodétecteur bénéficie ainsi d'un dispositif de filtrage angulaire qui lui est propre.

Chaque photodétecteur ne reçoit qu'une partie des rayons lumineux provenant du canal, cette partie étant comprise dans une région limitée par des angles d'incidence limites des rayons lumineux, définis par le dispositif de filtrage angulaire du photodétecteur.

Cela permet d'éviter que des rayons lumineux présentant un angle d'incidence supérieur aux angles d'incidence limites définis par le dispositif de filtrage angulaire, ne soient détectés par le photodétecteur.

Cette région limitée par les angles d'incidence limites correspond au cône de collecte du dispositif de filtrage angulaire.

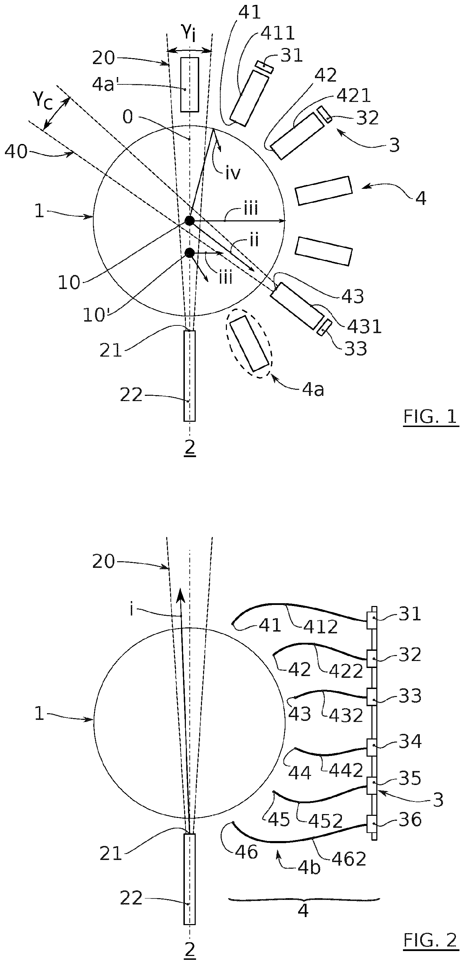

Ce cône de collecte 40 ainsi que le principe du filtrage angulaire est illustré en figure 1.

Dans le cadre du développement de la présente invention, il s'est avéré que le flou angulaire que l'on constate dans les détecteurs de l'art antérieur est notamment dû à des diffusions parasites qui sont générées par la présence de plusieurs particules 10, 10' dans le canal 1 ou encore par la variation de position d'une particule diffusante dans le canal 1.

En effet, avec les détecteurs de l'art antérieur, si un même photodétecteur 33 reçoit simultanément les rayons lumineux diffusés ii et iii respectivement des particules 10 et 10' présentes au même instant dans le canal 1, ce photodétecteur 33 n'est alors pas en mesure de fournir une information précise pour l'une seulement des particules, la particule 10 par exemple.

Avec le détecteur selon invention, le rayon lumineux iii de la particule 10' reste en dehors du cône de collecte 40 du dispositif de filtrage angulaire 431 associé au photodétecteur 33. Ce rayon lumineux iii n'est donc jamais détecté par le photodétecteur 33, bien que la particule 10' soit présente dans le canal. Il est alors possible d'analyser avec une précision significativement améliorée la particule 10 dont le rayon lumineux ii, entre dans le cône de collecte 40 et parvient au photodétecteur 33.

Le flou angulaire du photodétecteur est avantageusement limité grâce au dispositif de filtrage angulaire de la présente invention.

Comme indiqué ci-dessus, le flou angulaire constaté avec les détecteurs de l'art antérieur peut également provenir de la variation de position d'une particule diffusante dans le canal 1.

Dans ce cas, une particule 10 diffuse, lorsqu'elle est située dans une position initiale, par exemple un rayon lumineux ii et un rayon lumineux iii. Le rayon lumineux ii est compris dans le cône de collecte 40 du dispositif de filtrage angulaire 431 associé au photodétecteur 33. Il est donc détecté par le photodétecteur 33. Le rayon lumineux iii est en dehors du cône de collecte 40 du dispositif de filtrage angulaire 431 associé au photodétecteur 33. Il n'est pas détecté par le photodétecteur 33.

Lorsque la particule 10 se déplace dans une position de particule 10', les solutions de photodétecteur connues conduisent à une détection du rayon lumineux iii par le photodétecteur 33. Cette première source de détection parasite génère un flou angulaire dans l'analyse angulaire des rayons diffusés par la particule 10, 10'.

Avec la présente invention, le rayon lumineux iii reste avantageusement en dehors du cône de collecte 40 du dispositif de filtrage angulaire 431 associé au photodétecteur 33. Le rayon lumineux iii n'est donc jamais détecté par le photodétecteur 33, bien que la particule se soit déplacée. Le flou angulaire du photodétecteur est avantageusement limité grâce au dispositif de filtrage angulaire de la présente invention.

Une autre source de détection parasite provient de rayons lumineux iv diffusés indirectement, par exemple par un bord du canal 1 ou par une couche de protection, par exemple un encapsulant des photodétecteurs. Ces rayons lumineux iv indirects sont également détectés par les photodétecteurs des détecteurs de l'art antérieur. Ils génèrent alors un bruit de mesure dans l'analyse angulaire des rayons diffusés par la particule 10.

Avec la présente invention, les rayons lumineux iv restent avantageusement en dehors du cône de collecte 40 du dispositif de filtrage angulaire 431 associé au photodétecteur 33. Ces rayons lumineux iv ne sont donc jamais détectés par le photodétecteur 33. Le bruit de mesure du photodétecteur est avantageusement limité grâce au dispositif de filtrage angulaire de la présente invention.

Ainsi, la présente invention présente un avantage particulier pour les détecteurs dans lesquels un canal ou une couche d'encapsulation génère inévitablement des diffusions parasites.

Avec le détecteur selon l'invention, les rayons lumineux provenant du canal sont avantageusement collectés sélectivement par un dispositif de filtrage angulaire, en fonction de leurs angles d'incidence et de la position de leur source d'émission ou de réémission, à condition qu'ils se trouvent dans le cône de collecte du dispositif de filtrage angulaire considéré.

De manière avantageuse mais uniquement optionnelle, le détecteur selon l'invention peut présenter certaines au moins des caractéristiques facultatives énoncées ci-après qui peuvent éventuellement être utilisées en association ou alternativement.

Chaque dispositif de filtrage angulaire comprend de préférence une entrée optique présentant une ouverture numérique inférieure à 0,3, de préférence inférieure à 0,2, et de préférence de l'ordre de 0,1.

L'ouverture numérique est proportionnelle au demi-angle d'ouverture γ, correspondant à l'angle d'incidence limite, relativement à l'axe optique de l'entrée du dispositif de filtrage angulaire considéré, au-delà duquel un rayon lumineux n'est plus collecté par cette entrée.

Ainsi, les rayons lumineux diffusés présentant une direction de propagation formant un angle d'incidence, relativement à l'axe optique de l'entrée considérée, supérieur à γ sont rejetés par ladite entrée.

Pour un fluide tel que l'air présentant un indice de réfraction environ égal à 1, une ouverture numérique inférieure à 0,3 correspond à un demi-angle d'ouverture γ inférieur à environ 17°.

Un système de filtrage angulaire comprenant de tels dispositifs de filtrage angulaire permet donc avantageusement de limiter, voire d'éliminer la diffusion parasite, c'est-à-dire la diffusion comprenant les rayons indirectement diffusés iv par des éléments autres que la (ou les) particule(s).

Par ailleurs, la collecte au niveau d'une entrée d'un rayon directement diffusé par une particule implique que cette particule se trouve dans le cône de collecte défini par l'ouverture numérique de ladite entrée du dispositif de filtrage angulaire.

Les rayons directement diffusés iii par une particule située en dehors de ce cône de collecte ne sont pas collectés par cette entrée.

Un tel système de filtrage angulaire permet donc avantageusement de limiter le flou angulaire.

La présente invention concerne aussi un système comprenant un tel détecteur, le système étant pris parmi :

- un système d'alarme anti-incendie,
- un système de détection d'incendie,
- un système d'analyse de la qualité d'un fluide tel que l'air ou l'eau,
- un système d'alarme anti-pollution,
- un système de détection de poudre d'explosifs,
- un système de détection d'espèces microbiologiques.

La présente invention concerne encore un procédé de fabrication d'un tel détecteur de particules tel que défini dans la revendication 10 et comprenant au moins les étapes suivantes :

- fournir un substrat,
- définir sur une face du substrat le canal et les dispositifs de filtrage angulaire entourant le canal,
- former le canal au travers du substrat pour ménager un passage du fluide,
- former les dispositifs de filtrage angulaire au niveau de la face du substrat,
- associer un photodétecteur à chaque dispositif de filtrage angulaire.

## BREVE DESCRIPTION DES FIGURES

Les buts, objets, ainsi que les caractéristiques et avantages de l'invention ressortiront mieux de la description détaillée des modes de réalisation de cette dernière qui sont illustrés par les dessins d'accompagnement suivants dans lesquels :

La **figure 1** est un schéma de principe, en vue du dessus, illustrant une particule illuminée par un cône d'illumination et diffusant dans un cône de collecte d'un système de filtrage angulaire selon un exemple de réalisation d'un détecteur.

La **figure 2** est un schéma de principe, en vue du dessus, d'un mode de réalisation alternatif à celui illustré en figure 1.

La **figure 3A** illustre schématiquement, selon une vue en coupe, un mode de réalisation d'un dispositif de filtrage angulaire comprenant un guide d'onde.

La **figure 3B** illustre schématiquement, selon une vue en coupe, un mode de réalisation d'un dispositif de filtrage angulaire comprenant un guide d'onde associé à une microbille.

La **figure 3C** illustre schématiquement, selon une vue en coupe, un mode de réalisation d'un dispositif de filtrage angulaire comprenant une microbille.

La **figure 4A** illustre, en vue du dessus, un mode de réalisation d'un détecteur dans lequel le système de filtrage angulaire comprend des pièges de lumière.

La **figure 4B** illustre le mode de réalisation du détecteur de la figure 4A en perspective avec un couvercle.

La **figure 4C** illustre, en vue du dessus, un mode de réalisation d'un détecteur selon l'invention, dans lequel les photodétecteurs sont disposés en alternance sur deux cercles concentriques.

La **figure 4D** est une autre illustration du mode de réalisation du détecteur de la figure 4A, montrant une répartition des photodétecteurs selon deux angles de répartition.

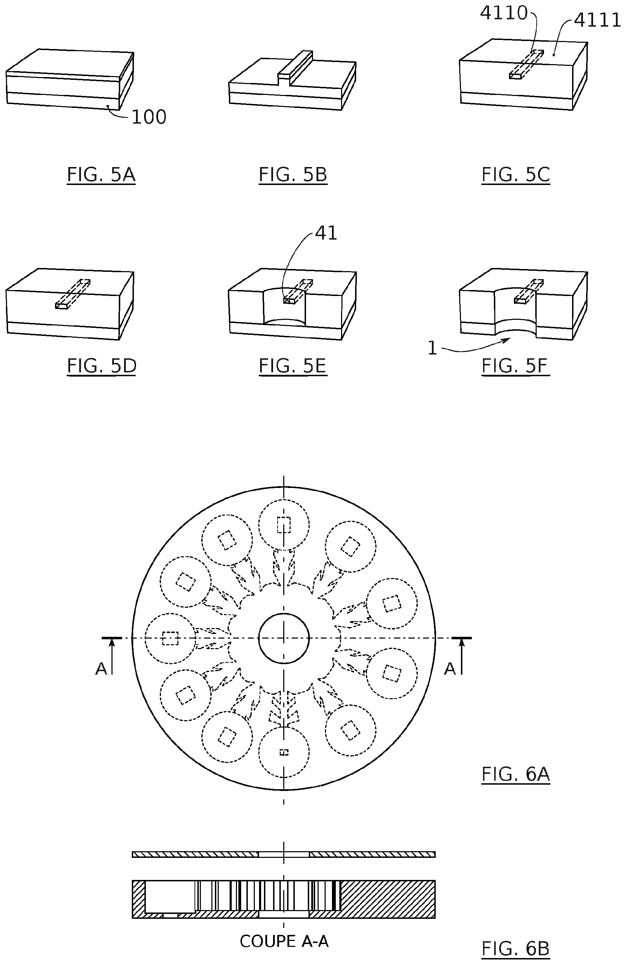

Les **figures 5A-5F** illustrent des étapes d'un procédé de fabrication pouvant convenir par exemple à la réalisation de détecteur selon les modes de réalisation illustrés en figures 1 à 3A. La **figure 5A** illustre un dépôt de couches destinées à former au moins un guide d'onde. La **figure 5B** illustre une formation d'au moins un guide d'onde par des techniques de lithographie/gravure. La **figure** 5C illustre une étape d'encapsulation d'au moins un guide d'onde. La **figure 5D** illustre une étape de recuit d'au moins un guide d'onde. La **figure 5E** illustre une étape de gravure superficielle d'un canal. La **figure 5F** illustre une étape de gravure profonde d'un canal.

La **figure 6A** illustre un mode de réalisation en demi-cercle d'un détecteur.

La **figure 6B** est une coupe transverse de la figure 6A selon A-A.

La **figure 7A** illustre schématiquement un échantillonnage angulaire symétrique réalisé par un détecteur selon un mode de réalisation de l'invention.

La **figure 7B** illustre schématiquement un échantillonnage angulaire asymétrique réalisé par un détecteur.

La **figure 8** illustre schématiquement un autre type d'échantillonnage angulaire réalisé par un détecteur.

Les dessins sont donnés à titre d'exemples et ne sont pas limitatifs de l'invention. Ces dessins sont des représentations schématiques et ne sont pas nécessairement à l'échelle de l'application pratique. En particulier, les dimensions relatives des différentes couches, motifs, cavité, photodétecteurs et autres structures ne sont pas représentatives de la réalité.

## DESCRIPTION DETAILLEE

L'ouverture numérique est une caractéristique d'un système optique, généralement notée NA selon la terminologie anglo-saxonne pour « Numerical Aperture ».

Elle est définie par $NA = n_0 . \sin i_0$, où $n_0$ est l'indice de réfraction dans le milieu d'observation, et $i_0$ est l'angle entre l'axe optique du système optique et le rayon le plus incliné par rapport à l'axe optique, qui sort du ou qui entre dans le système optique. Cet angle est appelé demi-angle d'ouverture. On notera $\gamma = 2.i_0$ l'angle d'ouverture.

Dans le cas des sources de lumière, il apparaît que les diodes électroluminescentes LED, peu directives, présentent des ouvertures numériques significativement plus grandes que les diodes lasers, très directives.

Dans le cas d'une structure de guidage telle qu'une fibre optique ou un guide d'onde comprenant un coeur d'indice ne et une gaine d'indice ng, l'ouverture numérique s'exprime selon la formule :

$$NA = \sin i_0 = \sqrt{n_c^2 - n_g^2}$$

indépendamment de l'indice de réfraction $n_0$ du milieu extérieur. NA est une caractéristique propre à la structure de guidage. Il apparaît qu'en diminuant le contraste d'indice, c'est-à-dire l'écart entre ces deux indices $n_c$ et $n_g$, l'ouverture numérique NA diminue.

Dans le cas d'une structure de guidage, le cône de collecte de la structure de guidage est fonction de l'ouverture numérique.

On entend par cône de collecte un faisceau de rayons lumineux, en particulier de rayons lumineux diffusés, admissibles par le système optique. Le cône de collecte ne présente pas obligatoirement une base circulaire ou une symétrie axiale. Le cône de collecte peut par exemple présenter une base elliptique ou une base rectangulaire.

Dans le cadre de la présente invention, le terme « particule » ou ses équivalents a pour définition un constituant d'un système physique considéré comme élémentaire par rapport aux propriétés étudiées.

Le terme particules désigne en particulier un objet solide, liquide ou solide mouillé en suspension dans l'air et dont la taille est microscopique. Par exemple, une particule est un élément de matière dont la plus grande dimension est inférieure à quelques millimètre ($10^{-3}$ mètres), de préférence à un millimètre, et de préférence à quelques dizaines de micromètres ($10^{-6}$ mètres) et de préférence inférieure au micromètre, voire de l'ordre du nanomètre ($10^{-9}$ m). Plus généralement, les particules présentent une taille supérieure à 40 Å ($10^{-10}$ m) et sont donc considérées comme optiquement continue. De manière générale, il s'agit d'objets composés de matière dont les dimensions sont petites par rapport aux dimensions de la cavité ou du canal de circulation des particules.

On entend par « taille » ou « diamètre » d'une particule la distance maximale entre deux points de la particule. Typiquement, on assimile une particule à un objet de géométrie sphérique, sa taille correspond donc au diamètre de la sphère.

On entend par un substrat, un guide d'onde, un film, une couche, « à base » d'un matériau A, un substrat, un guide d'onde, un film, une couche comprenant ce matériau A uniquement ou ce matériau A et éventuellement d'autres matériaux, par exemple des éléments dopants ou des éléments d'alliage. Ainsi, si un guide d'onde est désigné comme étant « à base de polymères », cela signifie qu'il peut être formé uniquement de polymères ou de polymères et éventuellement d'autres matériaux, par exemple un oxyde inorganique.

Il est précisé que dans le cadre de la présente invention, le terme « sur », « surmonte », « recouvre » ou « sous-jacent » ou leurs équivalents ne signifient pas « au contact de ». Ainsi par exemple, le dépôt d'une première couche sur une deuxième couche, ne signifie pas obligatoirement que les deux couches sont directement au contact l'une de l'autre mais cela signifie que la première couche recouvre au moins partiellement la deuxième couche en étant soit directement à son contact, soit en étant séparée d'elle par au moins une autre couche ou au moins un autre élément.

Sauf indication spécifique du contraire, des caractéristiques techniques décrites en détail pour un mode de réalisation donné peuvent être combinées aux caractéristiques techniques décrites dans le contexte d'autres modes de réalisation décrits à titre exemplaire et non limitatif.

Dans ce qui suit, le terme « absorption » ou ses équivalents se réfère au phénomène par lequel l'énergie d'une onde électromagnétique est transformée en une autre forme d'énergie, par exemple sous forme de chaleur. Dans la présente description, un matériau est considéré comme absorbant dès lors qu'il absorbe au moins 50% d'un rayonnement lumineux, de préférence au moins 75% et avantageusement au moins 90%. Il peut être caractérisé par un facteur d'absorption compris entre 0 et 1.

Dans ce qui suit, le terme « diffusion » ou ses équivalents se réfère au phénomène par lequel un milieu de propagation produit une répartition, dans de nombreuses directions, de l'énergie d'une onde électromagnétique, lumineuse par exemple.

Dans ce qui suit, le terme « réflexion » ou ses équivalents se réfère au phénomène de réémission depuis un élément ou une surface d'un rayonnement lumineux incident. Dans la présente description, un élément est considéré comme réfléchissant dès lors qu'il réémet une partie au moins d'un rayonnement lumineux incident, cette partie étant supérieure ou égale à 50%. Il peut être caractérisé par un facteur de réflexion compris entre 0 et 1.

Les termes « sensiblement », « environ », « de l'ordre de » signifient « à 10% près » ou, lorsqu'il s'agit d'une orientation angulaire, « à 5° près ». Ainsi, une direction sensiblement normale à un plan signifie une direction présentant un angle de $90 \pm 5°$ par rapport au plan.

Avant d'entamer une revue détaillée de modes de réalisation de l'invention, sont énoncées ci-après des caractéristiques faisant partie de l'invention ou optionnelles, qui peuvent éventuellement être utilisées en association ou alternativement :

-   Selon l'invention, chaque dispositif de filtrage angulaire comprend une entrée optique, l'entrée optique présentant une ouverture numérique inférieure à une ouverture numérique du photodétecteur auquel le dispositif de filtrage angulaire est associé.
-   Selon un exemple, le dispositif de filtrage angulaire est configuré pour définir un cône de collecte au niveau de son entrée optique, chaque cône de collecte présentant un angle d'ouverture $\gamma_c$ inférieur à 35°, de préférence inférieur à 25°, et de préférence compris entre 15° et 10°, de façon à collecter des rayons lumineux diffusés ayant des directions de propagation présentant des angles d'incidence, relativement à un axe optique de l'entrée optique considérée, inférieurs à 17,5°, de préférence inférieurs à 12,5°, et de préférence compris entre 7,5° et 5°.
-   Selon l'invention, le canal est cylindrique et les entrées optiques sont réparties en partie au moins autour du canal, selon une portion au moins d'au moins un cercle, et de préférence selon au moins un cercle complet, l'au moins un cercle étant de préférence centré sur un axe du canal.
-   Selon l'invention, les photodétecteurs sont disposés le long d'au moins deux cercles concentriques, en alternance. Ce mode de réalisation présente pour avantage d'être faiblement encombrant.
-   Selon un exemple, la pluralité de photodétecteurs comprend au moins 12 photodétecteurs.
-   Selon un exemple, les entrées optiques sont réparties le long d'un demi-cercle.
-   Selon un exemple, les photodétecteurs sont répartis le long d'un demi-cercle.
-   Selon un exemple, les photodétecteurs sont répartis le long d'une barrette.
-   Selon un exemple, chaque entrée optique présente un axe optique coïncidant avec un rayon du cylindre formant le canal.
-   Selon un exemple, le dispositif de filtrage angulaire comprend, entre son entrée et le photodétecteur qui lui est associé, une structure optique de guidage parmi une fibre optique, un guide d'onde, une lentille, un piège à lumière.
-   Selon un exemple, les photodétecteurs comprennent des surfaces de photodétection disposés de sorte à être situées dans un plan transverse à un axe longitudinal du canal, le détecteur comprenant en outre au moins une structure optique d'intégration configurée pour transmettre les rayons lumineux diffusés collectés au niveau d'une entrée optique, jusqu'à la surface de photodétection du photodétecteur associé à l'entrée optique considérée, ladite structure optique d'intégration étant par exemple une sphère intégrante, une rondelle intégrante, un prisme. Ce mode de réalisation présente pour avantage de permettre une intégration aisée des photodétecteurs lorsque le détecteur est réalisé par les techniques conventionnelles de microélectronique en particulier par des techniques planaires.
-   Selon un exemple, le canal est cylindrique et les entrées optiques sont réparties autour du canal sur des axes en prolongement de rayons du cylindre, deux axes successifs étant séparés par un angle de répartition θ compris entre 15° et 45°, de préférence de l'ordre de 30°.
-   Selon un exemple, l'angle de répartition θ des entrées optiques autour du canal est constant. Par exemple l'angle de répartition θ des entrées optiques autour du canal est constant le long du ou des cercles de détection. Cela permet de réaliser un échantillonnage angulaire symétrique. Ainsi, les entrées optiques sont réparties de manière symétrique autour de l'axe du canal. Selon un mode de réalisation alternatif, l'angle de répartition θ des entrées optiques autour du canal n'est pas constant. Il varie autour du canal. Typiquement, il varie le long du ou des cercles selon lesquels les entrées optiques sont positionnées. Ainsi, les entrées optiques sont réparties de manière non symétrique autour de l'axe du canal.

Cela permet d'augmenter la résolution angulaire du détecteur.

- Selon un exemple, une première partie des entrées optiques est répartie sur une première moitié de cercle et présente un premier angle de répartition θ1, par exemple de l'ordre de 30°, et une deuxième partie des entrées optiques est répartie sur une deuxième moitié de cercle et présente un deuxième angle de répartition θ2 différent de θ1, par exemple de l'ordre de 36°, de façon à réaliser un échantillonnage non redondant des rayons lumineux diffusés.
- Selon un exemple, le détecteur comprend en outre, pour au moins certains dispositifs de filtrage, une structure optique intermédiaire entre le canal et l'entrée optique, la structure optique intermédiaire étant configurée pour réduire la divergence du cône de collecte et/ou élargir une section du cône de collecte de l'entrée optique, ladite structure optique intermédiaire étant par exemple prise parmi une microlentille, une microbille.
- Selon un exemple, la source de lumière est isotrope et le détecteur comprend un dispositif de formation d'un faisceau incident associé à la source et présentant une sortie optique, ledit dispositif de formation d'un faisceau incident étant configuré pour former au niveau de la sortie optique un faisceau de rayons lumineux incidents (i) présentant un angle d'ouverture inférieur à 30°, de préférence inférieur à 20°, et de préférence de l'ordre de 10°, ledit dispositif de formation d'un faisceau incident comprenant de préférence l'un parmi une fibre optique, un guide d'onde, une lentille, un piège à lumière.
- Selon un exemple, le détecteur présente une dimension d'extension principale inférieure à 10 mm, de préférence de l'ordre de 5 mm, la dimension d'extension principale étant de préférence prise selon une direction transverse à un axe longitudinal du canal.
- Selon un exemple, le système de filtrage angulaire comprend une pluralité de pièges à lumière disposés autour du canal, chaque piège à lumière de cette pluralité de pièges à lumière étant configuré pour transmettre des rayons lumineux selon un faisceau de faible divergence, présentant par exemple un angle de divergence inférieur à 15°.
- Selon un exemple, chaque piège à lumière comprend un axe optique principal le long duquel les rayons lumineux présentant un angle de divergence relativement à l'axe optique inférieur à 15° sont transmis, et des branches absorbantes de part et d'autre de l'axe optique principal dans lesquelles les rayons lumineux présentant un angle de divergence relativement à l'axe optique supérieur à 15° sont absorbés.
- Selon un exemple de réalisation du procédé, les dispositifs de filtrage angulaire sont des pièges à lumière formés par gravure du substrat à partir de la face du substrat.

- Selon un exemple de réalisation du procédé, le procédé de fabrication du détecteur comprend en outre la formation d'une structure optique d'intégration configurée pour transmettre les rayons lumineux depuis le dispositif de filtrage angulaire jusqu'à une surface de photodétection du photodétecteur associé au dispositif de filtrage angulaire considéré, ladite surface de photodétection s'étendant principalement selon des plans parallèles à la face du substrat, et la structure optique d'intégration étant par exemple une rondelle intégrante gravée à partir de la face du substrat.
- Selon un exemple de réalisation du procédé, les photodétecteurs sont disposés sur une barrette, et la barrette de photodétecteurs est associée aux dispositifs de filtrage angulaire.

La présente invention trouve pour domaine préférentiel d'application la détection de particules de diverses tailles, de préférence dans le domaine des particules microscopiques, voire nanométriques. Par exemple la présente invention peut servir à la détection de particules issues de fumées, de poudre d'explosifs, de particules polluantes, de particules de poussière, de particules d'allergènes telles que les pollens, les spores de moisissure, ou encore de particules cancérigènes, ou des particules biologiques telles que des bactéries, des virus, ou encore des exosomes.

La présente invention s'applique à tout type de particules véhiculées par un fluide, que celui-ci soit liquide et/ou gazeux.

Le fluide présent ou s'écoulant dans le canal est par exemple de l'air. Tel est le cas pour les détecteurs intégrés dans les systèmes suivants : un système d'alarme anti-incendie, un système de détection d'incendie, un système de détection de poudre d'explosifs, un système d'analyse de la qualité d'un fluide tel que l'air, un système d'alarme anti-pollution.

Alternativement, le fluide peut être un liquide tel que de l'eau. Tel est le cas pour les détecteurs intégrés dans les systèmes de détection d'espèces microbiologiques.

La présente invention vise en particulier à réaliser des mesures de diffusion résolues angulairement, d'une ou de plusieurs particules, au sein d'un détecteur fabriqué à l'aide des technologies classiques de microfabrication.

Un premier exemple simplifié de détecteur va maintenant être décrit en référence à la figure 1, pour en comprendre le principe de fonctionnement.

Comme illustré sur ce schéma, le détecteur comprend un canal 1 dans lequel un fluide comprenant au moins une particule 10 peut circuler et un système de détection 3 comprenant une pluralité de photodétecteurs 31, 32, 33 destinés à collecter des rayons lumineux diffusés ii. Cette pluralité de photodétecteurs peut comprendre au moins sept photodétecteurs, de préférence au moins douze photodétecteurs, et de préférence un nombre de photodétecteurs supérieur à douze, de façon à ce que le détecteur puisse réaliser un échantillonnage angulaire suffisamment complet. La résolution angulaire du détecteur peut être augmentée en augmentant le nombre de photodétecteur.

Le détecteur est couplé à une source optique 2. La source 2 est de préférence distincte du détecteur. Cela permet de changer ou fabriquer la source indépendamment du détecteur.

Avantageusement, le détecteur comprend en outre un système de filtrage angulaire 4 configuré pour filtrer la collecte des rayons lumineux diffusés ii selon certains angles de diffusion seulement. Un tel système 4 permet de réduire le bruit de mesure dû aux diffusions parasites qui ne sont pas directement issues de la particule 10. Un tel système 4 permet également de réduire le flou angulaire généré par le mouvement de la particule 10, 10'. Ce système 4 permet également de réduire le flou angulaire généré par la diffusion sur un même photodétecteur, par exemple le photodétecteur 33, de rayons diffusés simultanément par plusieurs particules 10, 10' présentes dans le canal (les références 10, 10' s'entendent soit de deux positions différentes d'une même particule, soit de deux particules distinctes, par souci de concision).

Les éléments structurels permettant de maintenir à distance le canal 1, la source 2, le système de détection 3 et le système de filtrage angulaire 4 ne sont pas illustrés sur ce schéma de principe.

Ces éléments sont par exemple une couronne, un barillet ou une plaque support évidée en son centre permettant au fluide de s'écouler dans le canal 1.

Dans ce premier exemple et dans les exemples suivants, le canal 1 est de préférence cylindrique. La circulation du fluide se fait principalement selon l'axe longitudinal de ce canal. Sur la figure 1, cet axe est perpendiculaire au plan de la feuille. Ce canal peut présenter une dimension transverse, par exemple un diamètre, comprise entre 100 $\mu$m et 3 mm.

L'émission des rayons lumineux incidents i et la collecte des rayons lumineux diffusés ii se font de préférence dans un plan transverse à l'axe longitudinal de ce canal 1.

L'émission des rayons lumineux incidents i se fait au niveau d'une sortie optique 21 associée à une source de lumière 2.

La source 2 peut être associée à un dispositif de formation d'un faisceau incident 22 configuré pour transmettre des rayons lumineux incidents i compris dans un cône d'illumination 20 s'étendant depuis la sortie optique 21.

Ce dispositif de formation d'un faisceau incident 22 peut être une structure de guidage telle qu'un guide d'onde, une fibre optique, un piège de lumière ou un système de microlentilles par exemple.

La sortie optique 21 présente donc une ouverture numérique NAi permettant d'émettre les seuls rayons lumineux incidents i compris dans son cône d'illumination 20.

L'ouverture numérique NAi est inférieure à 0.3, de préférence inférieure à 0.2, et préférentiellement de l'ordre de 0.1. Ces gammes de valeurs de NAi correspondent environ à des angles d'ouverture $\gamma$i du cône d'illumination 20 inférieurs à 30°, de préférence inférieurs à 20°, et préférentiellement de l'ordre de 10°.

Selon un mode de réalisation, la source 2 est une diode laser permettant d'obtenir directement la directivité et la divergence souhaitées pour les rayons lumineux incidents i.

Selon un autre mode de réalisation, la source 2 est une diode LED associée à une structure de guidage permettant d'obtenir indirectement la directivité et la divergence souhaitées pour les rayons lumineux incidents i.

Le cône d'illumination 20 peut donc être formé directement à partir d'une diode laser ou indirectement à partir d'une LED.

Selon un mode de réalisation alternatif, la source 2 est le soleil.

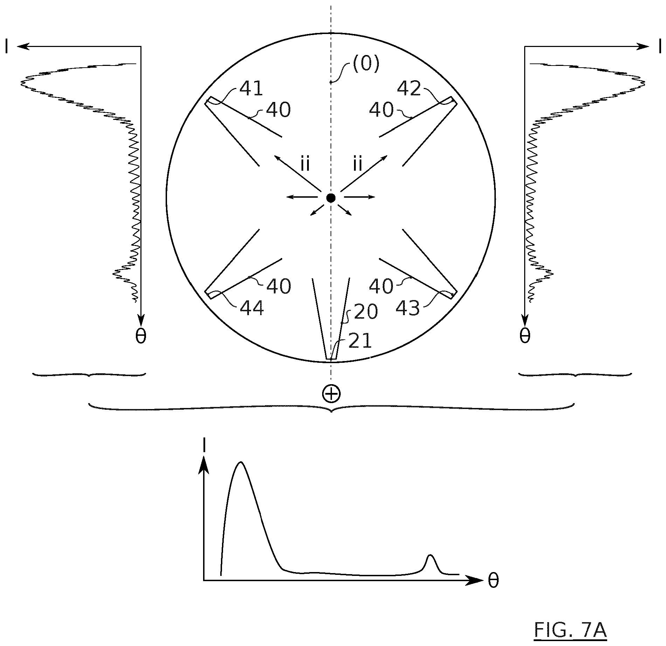

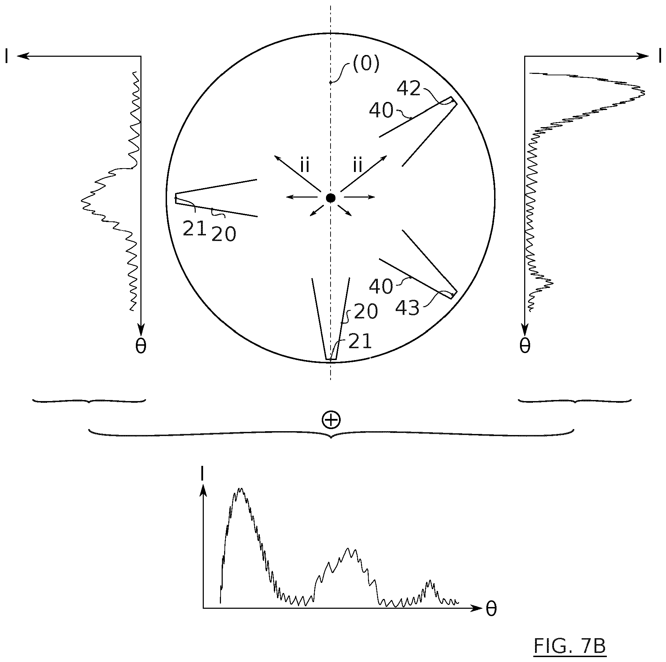

Une particule présente dans le canal 1 et passant dans le cône d'illumination 20 va générer des rayons lumineux diffusés ii. Ces rayons lumineux diffusés ii sont réémis par la particule 10 symétriquement de part et d'autre de l'axe optique (O) du cône d'illumination 20, également dénommé axe optique de la source 2, comme représenté aux figures 7A et 7B.

La collecte des rayons lumineux diffusés ii se fait avantageusement au niveau des entrées optiques 41, 42, 43 de dispositifs de filtrage angulaire 4a.

Comme cela sera illustré en détail par la suite, chaque dispositif de filtrage angulaire 4a est associé à un photodétecteur 31, 32, 33 et permet de filtrer angulairement les rayons lumineux parvenant à ce photodétecteur 31, 32, 33.

Dans la suite, un dispositif de filtrage angulaire 4a, 4b, 4c, 4d est formé de préférence par une entrée optique 4x et une structure de guidage 4xy.

Les entrées optiques 41, 42, 43 de ces dispositifs de filtrage angulaire sont disposées autour du canal 1, de façon à collecter des rayons lumineux diffusés ii présentant différents angles d'incidence.

Elles peuvent être disposées sur un demi-cercle, depuis la source 2 jusqu'à une entrée optique diamétralement opposée à la source 2, en regard de la source 2.

Elles peuvent alternativement être disposées sur un cercle complet. Elles peuvent également être disposées sur deux cercles concentriques, en alternance ou en quinconce, comme illustré dans la suite par d'autres exemples de réalisation. Ces choix pourront dépendre des coûts de fabrication, de l'encombrement et de la résolution angulaire souhaitée pour le détecteur.

Les entrées optiques peuvent délimiter en partie au moins le canal 1, par exemple directement au niveau d'une paroi du canal 1.

Alternativement, elles peuvent être séparées de la paroi du canal 1. Dans ce cas, elles peuvent être recouvertes par une couche de protection ou d'encapsulation de manière à éviter un encrassement desdites entrées optiques.

Le détecteur comprend de préférence un dispositif de filtrage 4a' dont l'entrée optique est alignée sur l'axe optique de la source 2, en regard de la source 2. Le photodétecteur associé à une telle entrée optique permet avantageusement de réaliser des mesures d'absorption des particules et/ou des contrôles de dérive de la source 2. Cette entrée optique peut également permettre de collecter une partie des rayons lumineux non utilisés pour l'analyse angulaire, par exemple des rayons lumineux incidents i non diffusés par la particule 10. Cette partie collectée peut ensuite être réinjectée dans le canal, soit de façon à maintenir une bonne luminosité du cône d'illumination 20, soit de façon à former un autre cône d'illumination, par exemple au niveau d'un étage inférieur du canal. Cela permet de limiter les pertes optiques du cône d'illumination 20. Cela permet in fine de bénéficier d'une majeure partie de la lumière émise par la source 2 aux fins d'analyse.

Les entrées optiques 41, 42, 43 transmettent de préférence les rayons lumineux diffusés ii aux photodétecteurs 31, 32, 33 par l'intermédiaire de structures de guidage.

Ces structures de guidage peuvent être par exemple des guides d'onde 411, 421, 431 comme illustré en figure 1. Comme cela sera décrit en détail en référence aux figures 2 à 4A, ces structures de guidage peuvent également être des fibres optiques 412, 422, 432, des systèmes de microlentilles 413 ou encore des pièges de lumière 414, 424, 434.

Une telle structure de guidage est configurée pour transmettre des rayons lumineux diffusés ii compris dans le cône de collecte 40 s'étendant depuis l'entrée optique associée à ladite structure de guidage.

Chaque entrée optique 41, 42, 43 présente donc une ouverture numérique NAc1, NAc2, NAc3, de préférence une même ouverture numérique NAc = NAc1 = NAc2 = NAc3, permettant de collecter les seuls rayons lumineux diffusés ii compris dans son cône de collecte 40.

L'ouverture numérique NAc est inférieure à 0.3, de préférence inférieure à 0.2, et préférentiellement de l'ordre de 0.1.

Ces gammes de valeurs de NAc correspondent environ à des angles d'ouverture $\gamma c$ du cône de collecte 40 inférieurs à 30°, de préférence inférieurs à 20°, et préférentiellement de l'ordre de 10°.

Pour améliorer la précision d'analyse angulaire du détecteur, il est préférable de réduire l'ouverture numérique NAc de la structure de guidage. Pour améliorer l'amplitude du signal lumineux collecté, il est préférable d'augmenter l'ouverture numérique NAc de la structure de guidage. Selon les performances souhaitées, l'homme du métier saura déterminer l'ouverture numérique NAc convenable, et réaliser les structures de guidage présentant une telle ouverture numérique NAc.

Le nombre d'entrées optiques et/ou de structures de guidage disposées autour du canal 1 est déterminé par l'échantillonnage angulaire souhaité. Par exemple, pour une répartition des entrées optiques et/ou des structures de guidage sur un demi-cercle, ce nombre est de préférence au moins égal à $\pi/2.\arcsin$ (NAc).

Tel qu'illustré à la figure 7A, pour une répartition symétrique des entrées optiques vis-à-vis de l'axe optique (O), l'échantillonnage angulaire réalisé par le détecteur est symétrique. L'échantillonnage angulaire est redondant et peut être moyenné. Le bruit affectant l'analyse angulaire du détecteur peut ainsi être réduit.

Tel qu'illustré à la figure 7B, pour une répartition asymétrique des entrées optiques vis-à-vis de l'axe optique (O), l'échantillonnage angulaire réalisé par le détecteur est asymétrique. L'échantillonnage angulaire est non redondant. La résolution angulaire du détecteur peut ainsi être augmentée.

Les différentes possibilités de répartition des entrées optiques autour du canal 1 sont compatibles avec tous les modes de réalisation décrits dans la suite. Par souci de concision, chacun des modes de réalisation illustrés dans la suite est décrit pour un type de répartition des entrées optiques uniquement, sans que cela soit limitatif.

L'intersection du cône d'illumination 20 et d'un cône de collecte 40 permet de définir un volume utile V tel qu'illustré à la figure 1. La superposition des volumes utiles V permet de définir un volume d'analyse dans lequel la diffusion de la particule 10 peut être analysée angulairement par le détecteur.

En réduisant l'angle d'ouverture des cônes d'illumination et/ou de collecte, le volume d'analyse est avantageusement réduit. Cela permet d'effectuer un filtrage spatial pour la position des particules. Cela permet de limiter le flou angulaire lors de l'analyse angulaire d'un flux de particules.

Selon un premier mode de réalisation illustré en figure 1, les structures de guidage associées aux entrées optiques 41, 42, 43 des dispositifs de filtrage angulaire 4a sont des guides d'ondes linéaires 411, 421, 431.

Ces guides d'ondes linéaires guident les rayons lumineux diffusés ii collectés aux entrées optiques correspondantes, principalement selon leurs axes longitudinaux respectifs.

Les axes longitudinaux de ces guides d'ondes linéaires coïncident avec des rayons du cylindre formant le canal 1.

Les photodétecteurs 31, 32, 33 (pour des raisons de clarté une partie seulement des photodétecteurs est représentée sur la figure 1) sont positionnés dans le prolongement des différents axes longitudinaux, de préférence de manière à ce que leurs surfaces de photodétection soient normales aux axes longitudinaux. Les photodétecteurs peuvent être couplés à leurs guides d'onde respectifs par couplage direct.

Pour un tel détecteur, la résolution angulaire du détecteur peut être assimilée à ± arcsin (NA).

L'efficacité de détection angulaire d'un tel détecteur est optimisée.

Selon un deuxième mode de réalisation illustré schématiquement à la figure 2, les structures de guidage associées aux entrées optiques 41, 42, 43 des dispositifs de filtrage angulaire 4b sont des fibres optiques 412, 422, 432.

Ces fibres optiques 412, 422, 432 permettent de guider les rayons lumineux diffusés ii collectés aux entrées optiques 41, 42, 43 correspondantes en particulier selon des trajets curvilignes.

Les axes optiques aux entrées 41, 42, 43 des fibres optiques 412, 422, 432 coïncident avec des rayons du cylindre formant le canal 1.

Les photodétecteurs 31, 32, 33 sont positionnés en sortie des fibres optiques 412, 422, 432, de préférence sur une même barrette linéaire, ou sur une même matrice de photodétecteurs.

Cela permet de simplifier l'intégration des photodétecteurs dans le détecteur. La barrette de photodétecteurs peut être couplée aux fibres optiques de manière à ce que les surfaces de photodétection des photodétecteurs soient normales aux axes optiques en sortie des fibres. Alternativement, la barrette de photodétecteurs peut être couplée aux fibres optiques par la tranche, i.e. de façon à ce que les surfaces de photodétection des photodétecteurs soient parallèles aux axes optiques en sortie des fibres.

Dans ce dernier cas, la sortie des fibres optiques peut présenter une interface orientée selon un angle de réflexion totale interne de façon à transmettre les rayons lumineux diffusés ii guidés aux surfaces de photodétection des photodétecteurs.

Alternativement, un réseau de couplage peut permettre d'extraire les rayons lumineux diffusés ii guidés dans les fibres, pour les transmettre aux surfaces de photodétection des photodétecteurs.

Les photodétecteurs peuvent être séparés des structures de guidage, de sorte qu'il n'y ait pas de contact entre les surfaces de photodétection des photodétecteurs et ces structures de guidage.

La barrette de photodétecteur du détecteur peut être facilement changée. La maintenance d'un tel détecteur est dès lors facilitée.

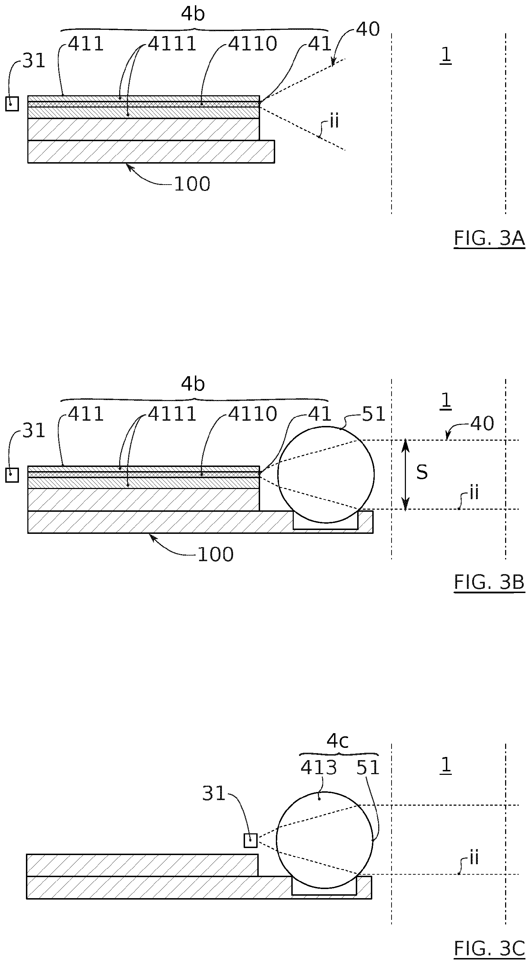

Les figures 3A, 3B, 3C illustrent des dispositifs de filtrage présentant différentes structures de collecte et/ou de guidage pouvant être associées aux photodétecteurs et/ou à la source de lumière 2.

La figure 3A illustre en coupe longitudinale un guide d'onde 411 comprenant un coeur 4110 et une gaine 4111, monté sur un support 100. Les rayons lumineux diffusés ii provenant du canal 1 et compris dans le cône de collecte 40 sont collectés à l'entrée optique 41, guidés par le guide d'onde 411 et transmis au photodétecteur 31.

Une telle structure de guidage formée par un guide d'onde est facilement réalisable à l'aide des technologies classiques de micro-fabrication.

La figure 3B illustre en coupe longitudinale un guide d'onde 411 comprenant un coeur 4110 et une gaine 4111, monté sur un support 100. Le support 100 est configuré pour recevoir en outre une structure optique intermédiaire 51 entre le canal 1 et l'entrée optique 41. Cette structure optique intermédiaire 51 contribue de préférence à la mise en forme du cône de collecte 40, par exemple de façon à améliorer la résolution angulaire et/ou la sensibilité de détection associée à l'entrée optique considérée.

Cette structure optique intermédiaire 51 peut être configurée pour réduire la divergence du cône de collecte 40, par exemple dans le cas d'un guide d'onde à fort contraste d'indice. Elle peut également élargir une section S à la base du cône de collecte 40, du côté de l'entrée 41. Une microbille 51, par exemple en un polymère transparent, présentant un diamètre compris entre 10 et 500 $\mu$m peut permettre une telle mise en forme du cône de collecte 40.

La figure 3C illustre en coupe longitudinale une bille, également désignée microbille, montée sur un support 100, directement associée à un photodétecteur 31. Cette bille est directement configurée pour former en partie au moins le dispositif de filtrage angulaire 4c. Dans cet exemple illustré en figure 3C, la bille forme à elle seul le dispositif de filtrage angulaire 4c. Cette bille présente donc une entrée optique 41 permettant de collecter les rayons lumineux diffusés ii compris dans le cône de collecte 40. Elle permet en outre de transmettre ces rayons lumineux diffusés ii au photodétecteur 31.

Une telle structure de collecte formée par une bille est avantageusement peu coûteuse.

Les entrées optiques associées à ces structures de collecte et/ou de guidage présentent de préférence une section S de collecte de quelques dizaines de $\mu$m², typiquement de l'ordre de 40 $\mu$m² voire de l'ordre de 100 $\mu$m².

La description des figures 3A-3C s'applique mutatis mutandis, selon le principe de retour inverse de la lumière, au cas d'une source de lumière 2, le photodétecteur 31 devenant la source 2, l'entrée 41 devenant la sortie 21, le cône de collecte 40 devenant le cône d'illumination 20, et les rayons lumineux diffusés ii devenant les rayons lumineux incidents i. Il est ainsi possible de focaliser le faisceau de source.

Les figures 5A à 5F décrivent schématiquement la réalisation d'un guide d'onde 411 d'un détecteur selon l'invention. Cette description peut également s'entendre de la réalisation d'une fibre optique. De manière plus générale, le principe illustré sur ces figures 5A à 5F peut servir à réaliser tout ou partie d'un détecteur selon les modes de réalisation illustrés aux figures 1 à 3B.

Un support 100 en matériau semi-conducteur tel que le silicium est fourni. Il peut être aminci au préalable de sorte à conserver une épaisseur de support 100 comprise entre 500 $\mu$m et 1 mm.

Une première couche en un premier polymère optique est déposée, de préférence sur une épaisseur comprise entre 5 μm et 50 μm, de façon à former la partie inférieure de la gaine 4111 du guide d'onde 411.

Une deuxième couche en un deuxième polymère optique est déposée, de préférence sur une épaisseur comprise entre 5 μm et 25 μm, de façon à constituer le coeur 4110 du guide d'onde 411 (figure 5A).

Ce deuxième polymère optique présente de préférence un indice de réfraction supérieur à celui du premier polymère optique, de façon à obtenir un confinement par contraste d'indices des rayons lumineux transmis par le guide d'onde 411.

Ce deuxième polymère optique est choisi de façon à présenter un faible contraste d'indice avec le premier polymère. En minimisant ainsi le contraste d'indice, l'ouverture numérique du guide d'onde est avantageusement minimisée.

Les épaisseurs de coeur 4110 et de gaine 4111 sont de préférence choisies de façon à ce que le guide d'onde 411 à faible contraste d'indice optique soit configuré pour guider la lumière se propageant selon des modes optiques dits étendus, présentant par exemple des dimensions d'extension transverse (par rapport à l'axe de propagation, ou encore par rapport à l'axe optique du guide d'onde) de plusieurs micromètres.

Le motif de coeur du guide d'onde 411 est ensuite défini et/ou gravé par les méthodes classiques de lithographie et/ou gravure (figure 5B). Selon un exemple, le deuxième polymère optique constituant le coeur est une résine optique photosensible négative de type SU-8 classiquement utilisée dans l'industrie de la microélectronique. La structuration du motif de coeur du guide d'onde peut dès lors se faire uniquement par lithographie, c'est-à-dire à partir d'une étape d'exposition, de préférence sous rayonnement UV, suivie d'une étape de développement, de préférence avec un développeur liquide.

Ce motif est ensuite encapsulé par une couche d'encapsulation à base du premier polymère de façon à former la partie supérieure de la gaine 4111 du guide d'onde 411, complémentaire de la partie inférieure (figure 5C). La partie supérieure présente de préférence une épaisseur sensiblement égale à l'épaisseur de la partie inférieure. Selon une possibilité alternative, cette couche d'encapsulation peut comprendre un troisième polymère optique présentant un indice de réfraction proche de ou sensiblement égal à celui du premier polymère optique.

La gaine 4111 est ainsi totalement formée.

De façon préférée mais optionnelle, une étape de recuit peut être effectuée de façon à générer une diffusion du premier et/ou du deuxième polymère optique au niveau des parois du coeur 4110 (figure 5D). Cette diffusion permet avantageusement d'améliorer la qualité optique des parois.

Le canal 1 du détecteur peut ensuite être gravé au travers des couches de gaine et/ou du coeur 4110.

Le canal 1 est gravé de façon à former l'entrée optique 41 du guide d'onde 411 en affleurement dudit canal 1 (figure 5E).

La formation du canal 1 est poursuivie par une gravure profonde du support 100 en silicium (figure 5F).

Ce procédé permet avantageusement de réaliser une pluralité de guides d'onde polymériques disposés dans un plan de base autour d'un canal 1 de façon peu coûteuse.

Selon une possibilité alternative, les guides d'ondes peuvent être à base de verres dopés à échange d'ions. Selon une autre possibilité, les guides d'ondes peuvent présenter un coeur à base de SiON et une gaine à base de SiO2.

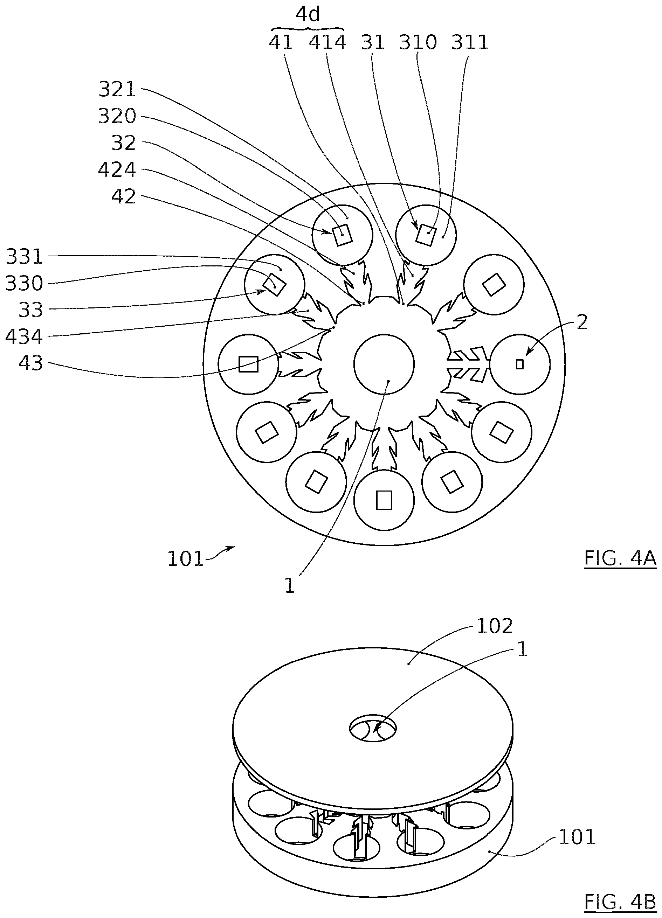

Selon un troisième mode de réalisation du détecteur illustré aux figures 4A à 4D, le détecteur se présente sous forme d'un barillet 101 s'étendant sur un plan de base (figure 4A). Ce barillet n'est cependant pas nécessairement cylindrique.

Ce barillet 101 comprend un canal 1 central traversant, de préférence cylindrique, une sortie optique 21 et une pluralité d'entrées optiques 41, 42, 43 disposés le long d'un premier cercle, dit cercle de collection, autour de et centré sur le canal 1. Le plan dans lequel s'étend principalement le barillet 101 est de préférence perpendiculaire à l'axe longitudinal du canal 1.

Le barillet 101 comprend en outre une pluralité de logements 311, 321, 331, de préférence cylindriques, disposés le long d'un deuxième cercle, dit cercle de détection, de diamètre supérieur au diamètre du cercle de collection. Le cercle de détection est de préférence centré sur le canal 1. Les cercles de détection et de collection sont de préférence concentriques.

Les logements 311, 321, 331 cylindriques sont destinés à recevoir la source de lumière 2 et les photodétecteurs 31, 32, 33.

Le barillet 101 comprend en outre une pluralité de pièges à lumière 414, 424, 434 disposés en étoile, selon des rayons du cercle de détection.

Cette pluralité de pièges à lumière forme le système de filtrage angulaire 4. Chaque piège à lumière 414, 424, 434 présente une entrée optique 41, 42, 43 et forme, en partie et de préférence entièrement un dispositif de filtrage 4d, comme cela est illustré en figure 4A.

Un piège à lumière est configuré pour transmettre des rayons lumineux selon un faisceau de faible divergence, présentant par exemple un angle de divergence inférieur à 15°.

Un tel piège à lumière peut comprendre un axe optique principal le long duquel les rayons lumineux présentant un angle de divergence relativement à l'axe optique inférieur à 15° sont transmis, et des branches absorbantes de part et d'autre de l'axe optique principal dans lesquelles les rayons lumineux présentant un angle de divergence relativement à l'axe optique supérieur à 15° sont absorbés.

Les branches absorbantes sont orientées selon le sens de propagation des rayons lumineux, en formant un angle aigu avec l'axe optique du piège à lumière. Elles sont configurées pour absorber les rayons lumineux se propageant en leur sein. Seuls les rayons lumineux se propageant principalement selon l'axe optique peuvent ainsi traverser le piège à lumière.

Le logement cylindrique abritant la source de lumière 2 est connecté à un piège à lumière « source » présentant une sortie optique 21 et présentant un axe principal orienté selon un rayon du cercle de collection.

Un logement 311, 321, 331 cylindrique abritant un photodétecteur 31, 32, 33 est connecté à un piège à lumière « photodétecteur » 414, 424, 434 présentant une entrée optique 41, 42, 43 et un axe principal orienté selon un rayon du cercle de collection.

Les branches absorbantes du piège à lumière « source » et les branches absorbantes des pièges à lumière « photodétecteur » sont orientées selon des sens opposés.

La lumière issue de la sortie 21 du piège à lumière « source » présente une faible divergence, quelle que soit la divergence de la source 2 proprement dite.

La lumière entrant dans les entrées optiques 41, 42, 43 est filtrée angulairement par les pièges à lumière « photodétecteur » 414, 424, 434, de sorte que seule une partie des rayons lumineux diffusés ii comprise dans le cône de collecte 40 de chacune des entrées optiques 41, 42, 43 parvient effectivement aux photodétecteurs 31, 32, 33 correspondants.

Un tel barillet 101 peut avantageusement être formé en un seul matériau, par exemple en silicium, sans qu'il soit nécessaire de recourir à des éléments optiques transparents pour filtrer et guider les rayons lumineux.

Les photodétecteurs 31, 32, 33 peuvent être disposés dans les logements correspondants de sorte que leurs surfaces de photodétection 310, 320, 330 respectives soient comprises dans un même plan parallèle au plan de base, sur un côté plan desdits logements cylindriques, par exemple au fond de ces logements 311, 321, 331.

La source 2 peut être disposée dans le logement correspondant de sorte que sa surface d'émission soit sensiblement comprise dans ce même plan parallèle au plan de base, au fond de son logement.

Une telle disposition facilite l'intégration des photodétecteurs et de la source dans le détecteur.

Les logements 311, 321, 331 cylindriques abritant les photodétecteurs 31, 32, 33 présentent de préférence des parois réfléchissantes permettant de réfléchir dans toutes les directions les rayons lumineux diffusés ii parvenant aux entrées des logements par l'intermédiaire des pièges à lumière correspondants, jusqu'aux surfaces de photodétection 310, 320, 330.

Le logement cylindrique abritant une source 2 à faible directivité, par exemple une LED, présente de préférence des parois réfléchissantes permettant de réfléchir dans toutes les directions les rayons lumineux émis par la source 2 jusqu'à la sortie du logement connectée au piège à lumière correspondant, de façon à former un faisceau de rayons lumineux incidents i. À cet effet, le logement cylindrique est recouvert d'une peinture réfléchissante et/ou diffusante de sorte à obtenir un éclairage relativement uniforme à l'intérieur du logement.

De tels logements cylindriques forment avantageusement des rondelles intégrantes 311, 321, 331 permettant d'améliorer respectivement la sensibilité de détection et la qualité d'illumination du détecteur à barillet.

Ces rondelles intégrantes 311, 321, 331 sont facilement réalisables par des techniques de fabrication planaires.

De façon alternative, les rondelles intégrantes peuvent être remplacées par des sphères intégrantes.

Le détecteur comprend également un couvercle 102 permettant de supprimer une lumière parasite extérieure (figure 4B).

Ce couvercle 102 est troué en son centre de façon à former un passage vers le canal 1.

La face du couvercle 102 en regard des logements cylindriques comprend de préférence des surfaces réfléchissantes au niveau de ces logements.

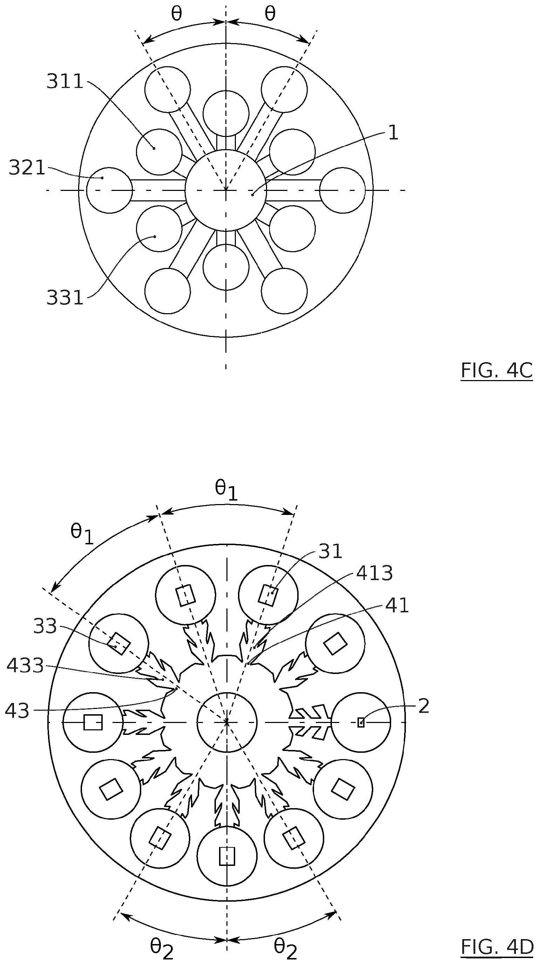

Selon une possibilité illustrée à la figure 4C, les logements cylindriques peuvent être disposés en quinconce le long de deux cercles de détection concentriques et de rayons différents. Les logements cylindriques 311, 321, 331 ne sont donc pas nécessairement à égale distance du canal 1. Ainsi, en effectuant une rotation centrée sur l'axe du canal 1 pour balayer les différents photodétecteurs, ces derniers sont disposés alternativement sur un cercle et sur l'autre.

Cette disposition permet d'optimiser l'encombrement lié aux logements cylindriques. Dès lors, l'angle de répartition θ entre deux photodétecteurs successifs peut être diminué. La résolution angulaire du détecteur peut être augmentée.

Ces avantages peuvent être encore renforcés si les photodétecteurs sont répartis sur plus de deux cercles, ou dans des plans différents.

Par exemple, les logements situés sur le premier cercle et les logements situés sur le deuxième cercle peuvent être situés de part et d'autre d'un plan médian perpendiculaire à l'axe longitudinal du canal 1. Dans ce cas les entrées optiques peuvent être centrées au niveau du plan médian, et des prismes, des structures de guidage et/ou des surfaces réfléchissantes peuvent renvoyer ou transmettre les rayons lumineux collectés vers les surfaces de photodétection des photodétecteurs en regard du plan médian.

L'angle de répartition θ peut être constant le long du ou des cercles de détection, en particulier entre deux photodétecteurs successifs disposés en dehors de l'axe optique (O). Cela permet de réaliser un échantillonnage angulaire symétrique par ce détecteur, tel qu'illustré à la figure 7A.

Alternativement, l'angle de répartition θ peut varier le long du ou des cercles de détection.

Selon une possibilité illustrée à la figure 4D, les logements cylindriques abritant les photodétecteurs peuvent être séparés par un premier angle de répartition θ1 sur une première moitié du cercle de détection, et par un deuxième angle de répartition θ2, différent de θ1, sur la deuxième moitié du cercle de détection. Selon un exemple le premier angle de répartition θ1 peut être de l'ordre de 30° et le deuxième angle de répartition θ2 peut être de l'ordre de 36°. Cela permet de réaliser un échantillonnage angulaire asymétrique par ce détecteur, tel qu'illustré à la figure 7B.

De façon générale, un tel échantillonnage asymétrique peut être réalisé en répartissant sur un cercle autour du canal 1 un nombre pair 2n d'entrées optiques du détecteur selon : 1 entrée optique en face de la source, n entrées optiques sur la première moitié du cercle, espacées d'un angle θ1 = π/(n+1) rad, et n-1 entrées optiques sur la deuxième moitié du cercle, espacées d'un angle θ2 = π/n rad.

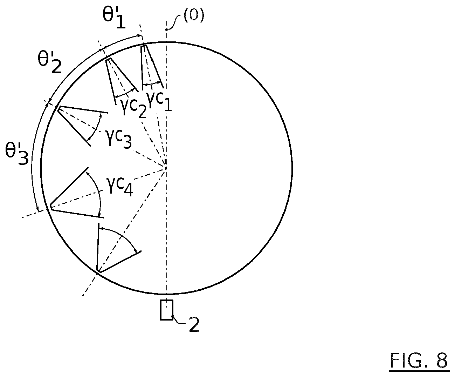

Selon une autre possibilité illustrée à la figure 8, les angles de répartition θ1', θ2', θ3'... et/ou les angles d'ouverture yc1, yc2, γc3, γc4... des cônes de collecte correspondants varient le long du ou des cercles de détection. Il est ainsi possible d'obtenir un premier type d'échantillonnage privilégiant la résolution angulaire, par exemple sur une première moitié ou sur un premier quart du cercle de détection sur un côté opposé à la source 2, et un deuxième type d'échantillonnage privilégiant la sensibilité, c'est-à-dire la réduction du bruit de mesure, par exemple sur une deuxième moitié ou sur un deuxième quart du cercle de détection sur un côté jouxtant la source 2.

Les figures 6A et 6B illustrent schématiquement la réalisation d'un détecteur à barillet.

Un support 100 en matériau semi-conducteur tel que le silicium est fourni. Il peut être aminci au préalable de sorte à conserver une épaisseur de support 100 comprise entre 500 μm et 1 mm, de préférence 750 μm.

Une première étape de lithographie/gravure permet de former les logements 311, 321, 331 cylindriques, les pièges à lumière 414, 424, 434 et une partie du canal 1.

La profondeur de gravure pour cette première étape peut être comprise entre 200 μm et 500 μm.

A l'issue de la première étape de gravure profonde, les logements 311, 321, 331 et le canal 1 ne sont pas entièrement percés.

Une deuxième étape de lithographie/gravure permet ensuite de percer les logements de façon à accueillir les photodétecteurs 31, 32, 33 et la source 2, et le canal 1 de façon à permettre le passage du fluide comprenant les particules.

Alternativement, le perçage peut se faire par micro usinage laser.

Les parois et les bases des rondelles intégrantes sont ensuite recouvertes de peinture réflective et diffusante, par exemple par sérigraphie ou par pulvérisation à l'aide d'un pochoir solide.

Le couvercle 102 peut être réalisé séparément, à partir d'un substrat silicium aminci par exemple.

Il est découpé aux dimensions du barillet 101, percé en son centre au niveau du canal 1, et recouvert de peinture réflective et diffusante sur les zones destinées à être en regard des logements du barillet 101.

Le barillet 101 et le couvercle 102 sont ensuite assemblés, par exemple par collage.

Selon une possibilité, le barillet et le couvercle sont réalisés au moins partiellement en matière plastique et l'assemblage du barillet et du couvercle est effectué par emboutissage à chaud. Selon une possibilité, l'assemblage est une soudure thermoplastique.

Selon une autre possibilité, le barillet et le couvercle sont réalisés par impression 3D. Ils peuvent être réalisés en une seule pièce. Dans ce cas, l'assemblage peut ne pas être nécessaire.

Au vu de la description qui précède, il apparaît clairement que l'invention propose une solution efficace pour améliorer la résolution angulaire du détecteur en limitant le flou angulaire et/ou le bruit de mesure.

L'invention n'est pas limitée aux modes de réalisation décrits mais s'étend à tout mode de réalisation entrant dans la portée de la revendication 1.

Par exemple, le détecteur peut comprendre plusieurs barillets superposés et/ou plusieurs sources de lumière. Il peut comprendre à la fois des structures de guidage de type guides d'onde et des structures de guidage de type pièges à lumière.

**Revendications**

1. Détecteur optique de particules comprenant au moins :

   - un canal cylindrique (1) destiné à recevoir un fluide comprenant au moins une particule (10) et à recevoir au moins un rayon lumineux incident (i);
   - un système de détection (3) comprenant une pluralité de photodétecteurs (31, 32, 33), chaque photodétecteur (31, 32, 33) étant apte à recevoir des rayons lumineux provenant du canal (1) et diffusés (ii) par l'au moins une particule (10),

   le détecteur comprenant en outre un système de filtrage angulaire (4) comprenant une pluralité de dispositifs de filtrage angulaire (4a, 4b, 4c, 4d) associés chacun à un photodétecteur (31, 32, 33), chaque dispositif de filtrage angulaire (4a, 4b, 4c, 4d) étant configuré pour filtrer angulairement les rayons lumineux (ii, iii, iv) provenant du canal (1) avant leur réception par le photodétecteur (31, 32, 33) auquel il est associé, chaque dispositif de filtrage angulaire (4a, 4b, 4c, 4d) comprenant une entrée optique (41, 42, 43) présentant une ouverture numérique inférieure à une ouverture numérique du photodétecteur (31, 32, 33) auquel le dispositif de filtrage angulaire (4a, 4b, 4c, 4d) est associé,

le détecteur étant **caractérisé en ce que** les entrées optiques (41, 42, 43) sont réparties en partie au moins autour du canal (1), selon une portion au moins d'au moins un cercle, les photodétecteurs (31, 32, 33) étant disposés le long d'au moins deux cercles concentriques de rayons différents, en quinconce.

2. Détecteur selon la revendication précédente, dans lequel l'entrée optique (41, 42, 43) de chaque dispositif de filtrage angulaire (4a, 4b, 4c, 4d) est inférieure ou égale à 0,3.

3. Détecteur selon l'une quelconque des revendications précédentes, dans lequel le dispositif de filtrage angulaire (4a, 4b, 4c, 4d) est configuré pour définir un cône de collecte (40) au niveau de son entrée optique (41, 42, 43), chaque cône de collecte (40) présentant un angle d'ouverture $\gamma_c$ inférieur à 35°, de façon à collecter des rayons lumineux diffusés (ii) ayant des directions de propagation présentant des angles d'incidence, relativement à un axe optique de l'entrée optique (41, 42, 43) considérée, inférieurs à 17,5°.

4. Détecteur selon l'une quelconque des revendications précédentes, dans lequel chaque entrée optique (41, 42, 43) présente un axe optique coïncidant avec un rayon du cylindre formant le canal (1).

5. Détecteur selon l'une quelconque des revendications précédentes, dans lequel le dispositif de filtrage angulaire (4a, 4b, 4c, 4d) comprend, entre son entrée (41) et le photodétecteur (31) qui lui est associé, une structure optique de guidage parmi une fibre optique (412, 422, 432), un guide d'onde (411, 421, 431), une lentille, ou un piège à lumière (413, 423, 433).

6. Détecteur selon l'une quelconque des revendications précédentes, dans lequel les photodétecteurs (31, 32, 33) comprennent des surfaces de photodétection (310, 320, 330) disposées de sorte à être situées dans un plan transverse à un axe longitudinal du canal (1), le détecteur comprenant en outre une structure optique d'intégration configurée pour transmettre les rayons lumineux diffusés (ii) collectés au niveau d'une entrée optique (41, 42, 43) jusqu'à la surface de photodétection (310) du photodétecteur (31) associé à l'entrée optique (41) considérée, ladite structure optique d'intégration étant par exemple une sphère intégrante, une rondelle intégrante (311, 321, 331), un prisme.

7. Détecteur selon l'une quelconque des revendications précédentes, dans lequel le canal (1) est cylindrique et les entrées optiques (41, 42, 43) sont réparties autour du canal (1) sur des axes en prolongement de rayons du cylindre, deux axes successifs étant séparés par un angle de répartition θ compris entre 15° et 45°, de préférence de l'ordre de 30°.

8. Détecteur selon la revendication précédente, dans lequel l'angle de répartition θ varie autour du canal (1) et dans lequel, de préférence, une première partie des entrées optiques (41, 42, 43) est répartie sur une première moitié de cercle et présente un premier angle de répartition θ1, par exemple de l'ordre de 30°, et dans lequel une deuxième partie des entrées optiques (41, 42, 43) est répartie sur une deuxième moitié de cercle et présente un deuxième angle de répartition θ2 différent de θ1, par exemple de l'ordre de 36°, de façon à réaliser un échantillonnage non redondant des rayons lumineux diffusés (ii).

9. Détecteur selon l'une quelconque des revendications précédentes comprenant en outre, pour au moins certains dispositifs de filtrage, une structure optique intermédiaire (51) entre le canal (1) et l'entrée optique (41, 42, 43), la structure optique intermédiaire (51) étant configurée pour réduire la divergence du cône de collecte (40) et/ou élargir une section du cône de collecte (40) de l'entrée optique (41, 42, 43), ladite structure optique intermédiaire (51) étant par exemple prise parmi une microlentille et une microbille.

10. Procédé de fabrication d'un détecteur de particules selon l'une quelconque des revendications 1 à 9, le procédé comprenant au moins les étapes suivantes :

- fournir un substrat,
- définir sur une face du substrat les dispositifs de filtrage angulaire (4a, 4b, 4c, 4d) entourant le canal (1),
- former le canal (1) au travers du substrat pour ménager un passage du fluide,
- former les dispositifs de filtrage angulaire (4a, 4b, 4c, 4d) au niveau de la face du substrat,
- associer un photodétecteur (31, 32, 33) à chaque dispositif de filtrage angulaire (4a, 4b, 4c, 4d).

11. Procédé selon la revendication précédente dans lequel les dispositifs de filtrage angulaire (4d) sont des pièges à lumière formés par gravure du substrat à partir de la face du substrat.

12. Procédé selon l'une quelconque des deux revendications précédentes, comprenant en outre la formation d'une structure optique d'intégration configurée pour transmettre les rayons lumineux depuis le dispositif de filtrage angulaire (4a, 4b, 4c, 4d) jusqu'à une surface de photodétection (310) du photodétecteur (31) associé au dispositif de filtrage angulaire (4a, 4b, 4c, 4d) considéré, ladite surface de photodétection (310) s'étendant principalement selon des plans parallèles à la face du substrat, et la structure optique d'intégration étant par exemple une rondelle intégrante (311, 321, 331) gravée à partir de la face du substrat.

**Patentansprüche**

1. Optischer Detektor für Partikel, umfassend mindestens:

   - einen zylindrischen Kanal (1) zum Empfangen eines Fluids, das mindestens ein Partikel (10) umfasst, und zum Empfangen mindestens eines einfallenden Lichtstrahls (i);
   - ein Erkennungssystem (3), das eine Vielzahl von Fotodetektoren (31, 32, 33) umfasst, wobei jeder Fotodetektor (31, 32, 33) Lichtstrahlen empfangen kann, die von dem Kanal (1) kommen und von dem mindestens einen Partikel (10) gestreut (ii) werden,

   der Detektor ferner ein Winkelfiltersystem (4) umfasst, das eine Vielzahl von Winkelfiltervorrichtungen (4a, 4b, 4c, 4d) umfasst, die jeweils mit einem Photodetektor (31, 32, 33) verbunden sind, wobei jede Winkelfiltervorrichtung (4a, 4b, 4c, 4d) so konfiguriert ist, dass sie die Lichtstrahlen (ii, iii, iv) aus dem Kanal (1) vor ihrem Empfang durch den Fotodetektor (31, 32, 33), mit dem sie verbunden ist, winkelmäßig filtert, wobei jede Winkelfiltervorrichtung (4a, 4b, 4c, 4d) einen optischen Eingang (41, 42, 43) mit einer numerischen Apertur umfasst, die kleiner als eine numerische Apertur des Photodetektors (31, 32, 33) ist, dem die Winkelfiltervorrichtung (4a, 4b, 4c, 4d) zugeordnet ist,
   wobei der Detektor **dadurch gekennzeichnet ist, dass** die optischen Eingänge (41, 42, 43) mindestens teilweise um den Kanal (1) herum verteilt sind, entlang mindestens eines Teils mindestens eines Kreises, wobei die Photodetektoren (31, 32, 33) entlang mindestens zwei konzentrischen Kreisen mit unterschiedlichen Radien versetzt angeordnet sind.

2. Detektor nach dem vorstehenden Anspruch, wobei der optische Eingang (41, 42, 43) jeder Winkelfiltervorrichtung (4a, 4b, 4c, 4d) kleiner oder gleich 0,3 ist.

3. Detektor nach einem der vorstehenden Ansprüche, wobei die Winkelfiltervorrichtung (4a, 4b, 4c, 4d) so konfiguriert ist, dass sie an ihrem optischen Eingang (41, 42, 43) einen Sammelkegel (40) definiert, wobei jeder Sammelkegel (40) einen Öffnungswinkel $\gamma_c$ von weniger als 35° aufweist, um gestreute Lichtstrahlen (ii) mit Ausbreitungsrichtungen mit Einfallswinkeln, relativ zu einer optischen Achse des betreffenden optischen Eingangs (41, 42, 43), von weniger als 17,5° zu sammeln.

4. Detektor nach einem der vorstehenden Ansprüche, wobei jeder optische Eingang (41, 42, 43) eine optische Achse aufweist, die mit einem Radius des Zylinders zusammenfällt, der den Kanal (1) bildet.

5. Detektor nach einem der vorstehenden Ansprüche, wobei die Winkelfiltervorrichtung (4a, 4b, 4c, 4d) zwischen ihrem Eingang (41) und dem ihr zugeordneten Photodetektor (31) eine optische Führungsstruktur umfasst, die aus einer optischen Faser (412, 422, 432), einem Wellenleiter (411, 421, 431), einer Linse oder einer Lichtfalle (413, 423, 433) besteht.

6. Detektor nach einem der vorstehenden Ansprüche, wobei die Photodetektoren (31, 32, 33) Photodetektoroberflächen (310, 320, 330) umfassen, die so angeordnet sind, dass sie sich in einer Ebene quer zu einer Längsachse des Kanals (1) befinden, wobei der Detektor ferner eine optische Integrationsstruktur umfasst, die so konfiguriert ist, dass sie die gestreuten Lichtstrahlen (ii), die an einem optischen Eingang (41, 42, 43) gesammelt werden, zu der Photodetektionsoberfläche (310) des Photodetektors (31) überträgt, der dem betreffenden optischen Eingang (41) zugeordnet ist, wobei die optische Integrationsstruktur zum Beispiel eine integrale Kugel, eine integrale Scheibe (311, 321, 331), ein Prisma ist.

7. Detektor nach einem der vorstehenden Ansprüche, wobei der Kanal (1) zylindrisch ist und die optischen Eingänge (41, 42, 43) um den Kanal (1) auf Achsen in Verlängerung der Radien des Zylinders verteilt sind, wobei zwei aufeinanderfolgende Achsen durch einen Verteilungswinkel $\theta$ zwischen 15° und 45°, vorzugsweise in der Größenordnung von 30°, getrennt sind.

8. Detektor nach dem vorstehenden Anspruch, wobei der Verteilungswinkel $\theta$ um den Kanal (1) herum variiert und wobei vorzugsweise ein erster Teil der optischen Eingänge (41, 42, 43) über eine erste Kreishälfte verteilt ist und einen ersten Verteilungswinkel $\theta 1$, zum Beispiel in der Größenordnung von 30°, aufweist, und wobei ein zweiter Teil der optischen Eingänge (41, 42, 43) über eine zweite Kreishälfte verteilt ist und einen zweiten Verteilungswinkel $\theta 2$ auf-

weist, der von θ1 unterschiedlich ist, zum Beispiel in der Größenordnung von 36°, um eine nicht-redundante Abtastung der gestreuten Lichtstrahlen (ii) zu erreichen.

9. Detektor nach einem der vorstehenden Ansprüche, umfassend ferner für mindestens einige Filtervorrichtungen eine optische Zwischenstruktur (51) zwischen dem Kanal (1) und dem optischen Eingang (41, 42, 43), wobei die optische Zwischenstruktur (51) so konfiguriert ist, dass sie die Divergenz des Sammelkegels (40) verringert und/oder einen Abschnitt des Sammelkegels (40) des optischen Eingangs (41, 42, 43) erweitert, wobei die optische Zwischenstruktur (51) beispielsweise aus einer Mikrolinse und einer Mikrokugel ausgewählt wird.

10. Verfahren zur Herstellung eines Detektors für Partikel nach einem der Ansprüche 1 bis 9, wobei das Verfahren mindestens die folgenden Schritte umfasst:

    - Bereitstellen eines Substrats,
    - Definieren der winkelförmigen Filtervorrichtungen (4a, 4b, 4c, 4d), die den Kanal (1) umgeben, auf einer Seite des Substrats,
    - Bilden des Kanals (1) durch das Substrat, um einen Durchgang für das Fluid zu ermöglichen,
    - Bilden der winkelförmigen Filtervorrichtungen (4a, 4b, 4c, 4d) an der Fläche des Substrats,
    - Verbinden eines Photodetektors (31, 32, 33) mit jeder Winkelfiltervorrichtung (4a, 4b, 4c, 4d).

11. Verfahren nach dem vorstehenden Anspruch wobei die winkelförmigen Filtervorrichtungen (4d) Lichtfallen sind, die durch Ätzen des Substrats von der Substratfläche aus gebildet werden.

12. Verfahren nach einem der beiden vorstehenden Ansprüche, das ferner die Bildung einer optischen Integrationsstruktur umfasst, die so konfiguriert ist, dass sie Lichtstrahlen von der Winkelfiltervorrichtung (4a, 4b, 4c, 4d) zu einer Photodetektionsoberfläche (310) des Photodetektors (31) überträgt, der der betreffenden Winkelfiltervorrichtung (4a, 4b, 4c, 4d) zugeordnet ist, die Photodetektionsoberfläche (310) sich hauptsächlich in Ebenen parallel zu der Substratfläche erstreckt, und die integrierende optische Struktur beispielsweise eine integrierende Scheibe (311, 321, 331) ist, die von der Substratfläche geätzt ist.

**Claims**

1. An optical particle sensor, comprising at least:

    - a cylindrical channel (1) intended to receive a fluid comprising at least one particle (10) and to receive at least one incident light ray (i);
    - a detection system (3) comprising a plurality of photodetectors (31, 32, 33), each photodetector (31, 32, 33) being able to receive light rays originating from the channel (1) and diffused (ii) by the at least one particle (10),

    the sensor further comprising an angular filtering system (4) comprising a plurality of angular filtering devices (4a, 4b, 4c, 4d) each associated with a photodetector (31, 32, 33), each angular filtering device (4a, 4b, 4c, 4d) being configured to angularly filter the light rays (ii, iii, iv) originating from the channel (1) before reception thereof by the photodetector (31, 32, 33) with which it is associated, each angular filtering device (4a, 4b, 4c, 4d) comprising an optical input (41, 42, 43) having a numerical aperture smaller than a numerical aperture of the photodetector (31, 32, 33) with which the angular filtering device (4a, 4b, 4c, 4d) is associated,

    the sensor being **characterised in that** the optical inputs (41, 42, 43) are distributed in part at least around the channel (1), over at least one portion of at least one circle, the photodetectors (31, 32, 33) being arranged along at least two concentric circles with different radii, in a staggered manner.

2. The optical sensor according to the preceding claim, wherein the optical input (41, 42, 43) of each angular filtering device (4a, 4b, 4c, 4d) is smaller than or equal to 0.3.

3. The optical sensor according to any one of the preceding claims, wherein the angular filtering device (4a, 4b, 4c, 4d) is configured to define a collection cone (40) at its optical input (41, 42, 43), each collection cone (40) having an aperture angle $\gamma_c$ smaller than 35°, so as to collect diffused light rays (ii) having directions of propagation having angles of incidence, relative to an optical axis of the considered optical input (41, 42, 43), smaller than 17.5°.

4. The sensor according to any one of the preceding claims, wherein the optical input (41, 42, 43) has an optical axis coinciding with a radius of the cylinder forming the channel (1).

5. The sensor according to any one of the preceding claims, wherein the angular filtering device (4a, 4b, 4c, 4d) comprises, between its input (41) and the photodetector (31) that is associated therewith, an optical guide structure amongst an optical fibre (412, 422, 432), a waveguide (411, 421, 431), a lens, or a light trap (413, 423, 433).

6. The sensor according to any one of the preceding claims, wherein the photodetectors (31, 32, 33) comprise photodetection surfaces (310, 320, 330) arranged so as to be located in a plane transverse to a longitudinal axis of the channel (1), the sensor further comprising an optical integration structure configured to transmit diffused light rays (ii) collected at an optical input (41, 42, 43) up to the photodetection surface (310) of the photodetector (31) associated with the considered optical input (41), said optical integration structure being for example an integrating sphere, an integrating washer (311, 321, 331), a prism.

7. The sensor according to any one of the preceding claims, wherein the channel (1) is cylindrical and the optical inputs (41, 42, 43) are distributed around the channel (1) on axes in line with radii of the cylinder, two successive axes being separated by a distribution angle θ comprised between 15° and 45°, preferably in the range of 30°.

8. The sensor according to the preceding claim, wherein the distribution angle θ varies around the channel (1) and wherein, preferably, a first portion of the optical inputs (41, 42, 43) is distributed over a first half of a circle and has a first distribution angle θ1, for example about 30°, and wherein a second portion of the optical inputs (41, 42, 43) is distributed over a second half of a circle and has a second distribution angle θ2 different from θ1, for example about 36°, so as to carry out a non-redundant sampling of the diffused light rays (ii).

9. The optical sensor according to any one of the preceding claims, further comprising, for at least some filtering devices, an intermediate optical structure (51) between the channel (1) and the optical input (41, 42, 43), the intermediate optical structure (51) being configured to reduce the divergence of the collection cone (40) and/or to enlarge a section of the collection cone (40) of the optical input (41, 42, 43), said intermediate optical structure (51) being, for example, selected amongst a microlens and a microbead.

10. A method for manufacturing an optical sensor according to any one of claims 1 to 9, the method comprising at least the following steps:

- providing a substrate,
- defining on one face of the substrate the angular filtering devices (4a, 4b, 4c, 4d) surrounding a channel (1),
- forming the channel (1) throughout the substrate to form a passage of the fluid,

- forming the angular filtering devices (4a, 4b, 4c, 4d) at a face of the substrate,
- associating a photodetector (31, 32, 33) with each of the angular filtering devices (4a, 4b, 4c, 4d).

11. The method according to the preceding claim, wherein the angular filtering devices (4d) are light traps formed by etching of the substrate starting from the face of the substrate.

12. The method according to any one of the preceding claims, further comprising forming an optical integration structure configured to transmit light rays from the angular filtering device (4a, 4b, 4c, 4d) up to a photodetection surface (310) of the photodetector (31) associated with the considered angular filtering device (4a, 4b, 4c, 4d), said photodetection surface (310) extending mainly on planes parallel to the face of the substrate, and the optical integration structure being for example an integrating washer (311, 321, 331) etched starting from the face of the substrate.

FIG. 1

FIG. 2

FIG. 3A

FIG. 3B

FIG. 3C

FIG. 4A

FIG. 4B

FIG. 4C

FIG. 4D

FIG. 5A

FIG. 5B

FIG. 5C

FIG. 5D

FIG. 5E

FIG. 5F

FIG. 6A

COUPE A-A

FIG. 6B

FIG. 7A

FIG. 7B

FIG. 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3062209 A1

- US 20050195605 A1

**Littérature non-brevet citée dans la description**

- **BOHREN ; HUFFMANN.** Absorption and scattering of light by small particles. Wiley and Sons, 1983